Europäisches Patentamt

European Patent Office

Office européen des brevets

(19)

(11) Veröffentlichungsnummer: **0 013 901**
**B1**

## EUROPÄISCHE PATENTSCHRIFT

(45) Veröffentlichungstag der Patentschrift:
12.01.83

(51) Int. Cl.³: **A 61 K 39/395, C 07 G 7/00**

(21) Anmeldenummer: 80100087.8

(22) Anmeldetag: 09.01.80

(54) Verfahren zur Herstellung einer für die intravenöse Applikation geeigneten Immunglobulinlösung, die IgM in ankonzentrierter Form enthält.

(30) Priorität: 18.01.79 DE 2901822

(43) Veröffentlichungstag der Anmeldung:
06.08.80 Patentblatt 80/16

(45) Bekanntmachung des Hinweises auf die Patenterteilung:
12.01.83 Patentblatt 83/2

(84) Benannte Vertragsstaaten:
AT BE CH DE FR GB IT LU NL SE

(56) Entgegenhaltungen:
DE-B-1 792 555
FR-A-1 370 553

CHEMICAL ABSTRACTS, Band 71, 1969, Zusammenfassung Nr. 47731v, Seite 187, Columbus, Ohio, US, W. STEPHAN: "Elimination of complement fixation in gamma-globulin by chemical modification with beta-propiolactone"

CHEMICAL ABSTRACTS, Band 78, Nr. 17, 30. April 1973; Zusammenfassung Nr. 109110x, Seite 314, Columbus, Ohio, US, A. VAN DER HOVEN et al.: "Isolation of immunogenically pure IgM from Cohn fraction III of pooled normal human plasma"

(73) Patentinhaber: Biotest-Serum-Institut GmbH, Flughafenstrasse 4, D-6000 Frankfurt-Niederrad (DE)

(72) Erfinder: Stephan, Wolfgang, Dr.-Dipl.-Chem., Philipp-Holzmann-Strasse 84, D-6072 Dreieich (DE)

(74) Vertreter: Beil, Walter, Dr. et al; BEIL, WOLFF & BEIL Rechtsanwälte Adelonstrasse 58, D-6230 Frankfurt am Main 80 (DE)

ACTORUM AG

Verfahren zur Herstellung einer für die intravenöse Applikation geeigneten Immunglobulinlösung, die IgM
in ankonzentrierter Form enthält

Die Erfindung betrifft ein Verfahren zur Herstellung einer für die intravenöse Applikation geeigneten Immunglobulinlösung durch Behandlung einer durch Fraktionierung aus Blutplasma oder Serum erhaltenen Proteinfraktion, die gegebenenfalls durch Behandlung mit kolloidaler Kieselsäure von Lipiden befreit wurde, mit β-Propiolacton bei Temperaturen von 20 bis 37 °C und pH-Werten von 7,0 bis 8,5 für die Dauer von 2 bis 10 Stunden bis zur pH-Konstanz, Aufarbeitung in bekannter Weise und Sterilfiltration, das dadurch gekennzeichnet ist, dass man als Ausgangsmaterial eine Plasma- oder Serumproteinfraktion verwendet, die IgM in ankonzentrierter Form enthält, und das Verhältnis von β-Propiolacton zu einer 5%igen Lösung der IgM enthaltenden Proteine auf 0,05 bis 0,15 ml pro 100 ml einstellt.

Das erfindungsgemäss erhältliche, für die intravenöse Anwendung geeignete IgM-haltige Immunglobulinpräparat weist eine hohe Antikörperaktivität gegen gramnegative und grampositive Bakterien auf.

Bei den bekannten Fraktionierungen von Blutplasma oder Serum, z.B. der Cohn'schen Alkoholfraktionierung von menschlichem Blutplasma oder der Rivanol (2-Ethoxy-6,9-diaminoacridin)-Ammonsulfat-Fraktionierung [z.B. Behringwerk-Mitteilungen 43, 162 (1964)] von Serum, werden bekanntlich u.a. auch Fraktionen erhalten, die IgM in ankonzentrierter Form enthalten. Dem derzeitigen Stand der Wissenschaft zufolge bewirkt jedoch eine durch derartige Fraktionierprozesse hervorgerufene Denaturierung am Fc-Teil des Antikörpermoleküls die sogenannte antikomplementäre Aktivität, die für die intravenöse Unverträglichkeit von fraktionierten Immunglobulinen verantwortlich ist. Diese antikomplementäre Aktivität wird auch durch das von van der Hoven et al. [Immunochemistry 10(2), 107–114 (1973) bzw. C.A. 78, 109 110x (1973)] beschriebene Verfahren zur Isolierung von hinsichtlich seiner Immunität reinem IgM aus einer Cohn-Fraktion III von menschlichem Blutplasma durch Fällung mit Polyethylenglykol oder Euglobulin-Fällung, Entfernung der Lipoproteine, Säulenchromatographie und Immunoadsorption nicht entfernt.

Für die Herstellung von intravenös verträglichen Immunglobulinpräparaten vom IgG-Typ, die vor allem Antikörper gegen Viren enthalten, wurde bereits eine Reihe von Verfahren entwickelt, z.B. ein Abbau mittels Pepsin [H.E. Schultze und G. Schwick: Dtsch. med. Wochenschrift, 87, 1643 (1962)], ein Abbau mittels Plasmin [S. Barandun et al.; Vox Sang. 28, 157 (1975)], ein Abbau mittels Salzsäure [S. Barandun et al.: Vox Sang. 7, 187 (1962)] oder eine chemische Modifizierung mittels β-Propiolacton [W. Stephan: Z. Klin. Chem. Klin. Biochem. 7, 282 (1969) bzw. C.A. 71, 477 31v (1969) oder DE-B-1 792 555]. Wenn in den zuletzt genannten Literaturstellen an vielen Stellen von «γ-Globulin» (in der Einzahl) gesprochen wird, so werden hier ersichtlich nicht Gammaglobuline allgemein beschrieben, sondern es handelt sich immer um Präparate, die der Cohn-Fraktion II oder dem Präzipitat II der Rivanol-Ammonsulfat-Fraktionierung entsprechen, d.h. um Standard-Gammaglobulin bzw. γG-Globulin bzw. 7Sγ-Globulin bzw. IgG. Dies wird deutlich aus der Versuchsbeschreibung der Rivanol-Ammonsulfat-Fraktionierung auf Seite 285 von Z. Klin. Chem. Klin. Biochem. 7 (1969), wonach das Filtrat der Rivanol-Fällung weiterverarbeitet wird. Dem Schema der Serumfraktionierung mit Rivanol und Ammoniumsulfat aus Behringwerk-Mitteilungen 43, 162 (1964) ist zu entnehmen, dass aus dem Filtrat der Rivanol-Fällung mit Ammoniumsulfat das Präzipitat II gefällt wird, das das 7Sγ-Globulin enthält, während aus dem Präzipitat I der Rivanol-Fällung nach Auflösen und Fällung mit Ammoniumsulfat das Präzipitat III gewonnen wird, das das IgM (γ₁-M-Globulin) enthält. Offensichtlich wird aber nach der Versuchsbeschreibung auf Seite 285 von Z. Klin. Chem. Klin. Biochem. 7 (1969) das Präzipitat I der Rivanol-Fällung verworfen. Weiterhin wird auf Seite 283 von Z. Klin. Chem. Klin. Biochem. 7 (1969) klargestellt, dass bei der vorstehenden Fällung als Hauptbestandteil 7Sγ-Globulin erhalten wird.

Die Versuchsbeschreibung zur Behandlung von komplementfixierendem γ-Globulin mit β-Propiolacton auf Seite 285 von Z. Klin. Chem. Klin. Biochem. 7 (1969) macht zwar keine näheren Angaben zu der verwendeten γ-Globulin-Lösung; ein Vergleich mit dem einzigen Beispiel der DE-B-1 792 555 lässt jedoch leicht erkennen, dass es sich in beiden Fällen um die gleiche Standard-Gammaglobulinlösung mit einer Proteinkonzentration von 7 bis 10% gehandelt haben muss.

Obwohl die verschiedenen vorstehenden Verfahren zur Herstellung intravenös verträglicher Immunglobulinpräparate vom IgG-Typ bereits seit einigen Jahren bekannt sind, gibt es bisher keine Methode, die es erlaubt, intravenös verträgliche hochgereinigte Immunglobulinpräparate herzustellen, die IgM in ankonzentrierter Form enthalten, wie es für die Bekämpfung bakterieller Infektionen notwendig ist, obwohl IgM-haltige Fraktionen, z.B. die Cohn-Fraktion III, seit der Entwicklung der Alkoholfraktionierung (1940–1950) zur Verfügung stehen. Die Tatsache, dass bisher trotzdem keine intravenös verträglichen IgM-Konzentrate bereitgestellt wurden, ist wahrscheinlich u.a. auf die unterschiedliche Molekülstruktur des IgM-Moleküls gegenüber dem IgG-Molekül zurückzuführen.

Eine Übertragung der bekannten Behandlung von Immunglobulinpräparaten vom IgG-Typ mit β-Propiolacton auf Immunglobulinpräparate, die IgM in ankonzentrierter Form enthalten, lag deshalb nicht nahe, weil der IgM-Antikörper pro Molekül 5 Fc-Teile enthält, während der IgG-Antikörper lediglich 1 Fc-Teil pro Molekül aufweist, und deshalb erwartet werden musste, dass für eine Behandlung IgM-haltiger Präparate mit β-Propiolacton nur eine so hohe Konzentration wirksam die antikomplementäre Aktivität beseitigen könnte, die andererseits gleichzeitig einen weitgehenden Verlust der Antikörperaktivität mit sich brächte. Diese Vorstellungen kamen praktisch einem Vorurteil gleich.

Nur mit diesem Vorurteil ist zu erklären, warum trotz des starken Bedürfnisses nach einem intravenös verträglichen Immunglobulinpräparat, das IgM in ankonzentrierter Form enthält, bis heute kein solches Präparat beschrieben oder erhältlich ist. Alle bisher beschriebenen IgM-Präparate, wie z.B. das aus Immunochemistry 10 (2), 107 (1973), sind nicht intravenös verträglich und können daher lediglich als Ausgangsmaterial für das erfindungsgemässe Verfahren eingesetzt werden. Derartige Ausgangsmaterialien standen aber bereits seit 1940 bis 1950 zur Verfügung. Das starke Bedürfnis nach einem intravenös verträglichen Immunglobulinpräparat, das IgM in ankonzentrierter Form enthält, beruht auf der hohen Antikörperaktivität von IgM gegen gramnegative und grampositive Bakterien, die besonders seit dem Auftreten von immer mehr Antibiotikaresistenz bei Bakterien an Bedeutung gewonnen hat. Die bisher bekannten intravenös verträglichen Immunglobulinpräparate vom IgG-Typ können dieses Bedürfnis nicht befriedigen, da sie vor allem Antikörper gegen Viren enthalten und nur in sehr geringem Masse wirksam gegen Bakterien sind. Ein entsprechender Vergleich der Antikörperaktivitäten wird nachstehend erläutert.

Überraschenderweise wurde nun gefunden, dass trotz Anhäufung der antikomplementär wirksamen Fc-Teile im IgM-Molekül dessen antikomplementäre Aktivität mit ähnlich geringen Mengen β-Propiolacton wie bei der Herstellung von intravenös verträglichem Immunglobulin vom IgG-Typ nach der DE-AS 1 792 555, nämlich mit 0,05 bis 0,15 ml β-Propiolacton pro 100 ml einer 5%igen Immunglobulinlösung, beseitigt werden kann.

Es gelingt somit im erfindungsgemässen Verfahren, Immunglobulinlösungen, die IgM in ankonzentrierter Form enthalten, unter weitgehendem Erhalt ihrer Antikörperaktivitäten intravenös verträglich zu machen.

Die erfindungsgemäss erhältliche, intravenös verträgliche Immunglobulinlösung, die IgM in ankonzentrierter Form enthält, ist frei von antikomplementärer Aktivität und enthält hohe Antikörperaktivitäten gegen bakterielle Erreger.

Bei der Durchführung des erfindungsgemässen Verfahrens wird eine IgM-haltige Proteinfraktion, die nach einem der bekannten Fraktionierungsverfahren aus Blutplasma oder Serum erhalten wurde, mit solchen Mengen β-Propiolacton behandelt, dass das Verhältnis von β-Propiolacton zu einer 5%igen Lösung der IgM enthaltenden Proteine 0,05 bis 0,15 ml pro 100 ml beträgt. Als Ausgangsmaterial können beispielsweise IgM-haltige Fraktionen der Cohn'schen Alkoholfraktionierung von menschlichem Blutplasma oder der Rivanol (2-Ethoxy-6,9-diaminoacridin)-Ammonsulfat-Frakionierung verwendet werden. Ein besonders bevorzugtes Ausgangsmaterial ist die Cohn-Fraktion III aus menschlichem Plasma. Eine derartige IgM-haltige Fraktion wird vorzugsweise zunächst in physiologischer (0,9%iger Kochsalzlösung zu einer etwa 5%igen Proteinlösung gelöst. Vor der Behandlung mit β-Propiolacton sollte diese Proteinlösung vorzugsweise durch Behandlung mit kolloidaler Kieselsäure von Lipiden befreit werden. Auch eine Behandlung mit Diethylamino-ethylgruppen tragenden, quervernetzten Dextranen oder Cellulose, vorzugsweise mit unter der Handelsbezeichnung DEAE-Sephadex A-50 oder DEAE-Cellulose bekannten Anionenaustauschern kann vorgenommen werden.

Wenn als Ausgangsmaterial beispielsweise Präzipitat III der Rivanol-Ammonsulfat-Fraktionierung eingesetzt wird, so kann man diesen Niederschlag zunächst in Wasser lösen und gegen eine Phosphatpufferlösung vom pH-Wert 6,2 dialysieren und sodann den dabei erhaltenen Niederschlag der Euglobulinfällung in physiologischer Kochsalzlösung lösen.

Die Behandlung mit β-Propiolacton wird sodann bei Temperaturen von 20 bis 37 °C und pH-Werten von 7,0 bis 8,5, vorzugsweise 8,0, für die Dauer von 2 bis 10 Stunden, vorzugsweise 4 bis 6 Stunden, bis zur pH-Konstanz durchgeführt.

Nach der Behandlung mit β-Propiolacton wird die erhaltene Lösung in bekannter Weise aufgearbeitet, beispielsweise sterilfiltriert. Es kann auch zunächst eine Behandlung mit Aktivkohle durchgeführt werden.

Die günstigen Eigenschaften der im erfindungsgemässen Verfahren erhältlichen Immunglobulinlösungen wurden durch die nachfolgenden Untersuchungen belegt.

1. Antikomplementäre Aktivität

| Produkt | Komplementverbrauch (ml Komplement [1:30]) pro 1 ml Probe |
|---|---|
| IgM-Konzentrat vor β-Propiolacton-Behandlung | 7 |
| IgM-Konzentrat nach β-Propiolacton-Behandlung (0,12 ml β-Propiolacton pro 100 ml) | maximal 0,3 |

Die antikomplementäre Aktivität entspricht den Werten für handelsübliche intravenös verträgliche Präparate vom IgG-Typ.

2. Beeinflussung von bakteriellen Antikörperaktivitäten durch
β-Propiolacton-Modifizierung

| Bakterientyp | Antikörperaktivitäten | | |
|---|---|---|---|
| | Ausgang | nach Behandlung mit 0,12 ml β-Propiolacton pro 100 ml Lösung (5%) | |
| | (Score = Reaktions- stärke) | (Score = Reaktions- stärke) | (%) |
| E. Coli | 36 | 38 | 105 |
| Klebsiellen | 37 | 26 | 70 |
| Pyocyaneus | 38 | 37 | 97 |
| Streptococcus viridans | 46 | 34 | 74 |
| Streptococcus haemolyticus | 40 | 30 | 75 |
| Enterokokken | 28 | 24 | 86 |
| Staphylokokken | 43 | 34 | 76 |

Score:    Summe der Agglutinationsstärken der einzelnen Stufen
einer geometrischen Verdünnungsreihe:

$$+ + + + \; = \; 10 \qquad\qquad + \; = \; 3$$
$$+ + + \; = \; 8 \qquad\qquad \pm \; = \; 1$$
$$+ + \; = \; 5$$

Die Antikörperaktivität nach β-Propiolacton-Behandlung bleibt weitgehend erhalten.

3. Vergleich der Antikörperaktivitäten eines handelsüblichen i.V. Immunglobulinpräparates mit dem erfindungsgemässen Präparat

| Präparat | Antikörperaktivitäten (Score-Werte) gegen | | | | | | |
|---|---|---|---|---|---|---|---|
| | E. Coli | Klebs. | Pyo. | Staph. | Strept. haemol. | Strept. virid. | Ente- rok. |
| handelsübliches i.V. Immunglobulin | 14 | 9 | 11 | 3 | 0 | 1 | 0 |
| erfindungsgemässes IgM-Konzentrat | 38 | 26 | 37 | 34 | 30 | 34 | 24 |

* erhalten durch chemische Modifizierung mit β-Propiolacton.

4. Vergleich der Immunglobulinzusammensetzung eines handelsüblichen i.V. Immunglobulinpräparates mit der eines erfindungsgemässen IgM-Konzentrates

| Präparat | IgG | IgA (mg %) | IgM | Gesamt- protein- Gehalt (g %) | antikomplementäre Aktivität (ml Komplement [1:30] pro 1 ml) |
|---|---|---|---|---|---|
| handelsübliches i.V. Immunglobulin* | 4900 | 100 | Spuren | 5,0 | 0,3 |
| erfindungsgemässes i.V. IgM-Konzentrat | 4000 | 500 | 500 | 5,0 | 0,3 |

* erhalten durch chemische Modifizierung mit β-Propiolacton.

Die folgenden Beispiele erläutern die Erfindung.

Beispiel 1

Cohn-Fraktion III aus menschlichem Plasma wurde in 0,9%iger Kochsalzlösung zu einer 5%igen Proteinlösung gelöst, mit 3% Aerosil von Lipiden befreit und anschliessend mit 80 mg DEAE-Sephadex A-50 pro 1 g Protein behandelt. Danach wurde die Lösung, die eine Proteinkonzentration von 5% aufwies, bei einem pH-Wert von 8,0 und bei 37°C mit 0,1 ml β-Propiolacton pro 100 ml Lösung bis zur pH-Konstanz behandelt. Die erhaltene Lösung wurde in bekannter Weise gegen 0,9%ige Kochsalzlösung dialysiert und sterilfiltriert und eignete sich sodann für die intravenöse Applikation.

Beispiel 2

Cohn-Fraktion III aus 2 Stunden auf 56 °C erhitztem menschlichem Plasma wurde in 0,9%iger Kochsalzlösung zu einer 5%igen Proteinlösung gelöst, mit 3% Aerosil von Lipiden befreit und anschliessend mit 80 mg DEAE-Sephadex A-50 pro 1 g Protein behandelt. Danach wurde die Lösung, deren Proteinkonzentration 5% betrug, bei einem pH-Wert von 8,0 und bei 37 °C mit 0,05 ml β-Propiolacton pro 100 ml Lösung bis zur pH-Konstanz behandelt. Die erhaltene Lösung wurde mittels Dialyse und Sterilfiltration in bekannter Weise aufgearbeitet und eignete sich sodann für die intravenöse Applikation.

Beispiel 3

Cohn-Fraktion III aus menschlichem Plasma wurde in 0,9%iger Kochsalzlösung zu einer 5%igen Lösung gelöst, mit 3% Aerosil von Lipiden befreit und mit 80 mg DEAE-Sephadex A-50 pro 1 g Protein behandelt. Danach wurde mit 0,05molarem Acetatpuffer auf 1,5% Protein verdünnt, auf pH 4,8 eingestellt und mit 1,5 ml Octansäure pro 100 ml Lösung sowie mit 0,4 g $Ca_3(PO_4)_2$ pro 100 ml Lösung behandelt. Nach dem Zentrifugieren und Dialysieren gegen 0,9%ige Kochsalzlösung wurde der Überstand ankonzentriert und bei einer Proteinkonzentration von 5% bei pH 8,0 und Raumtemperatur (20 bis 25 °C) mit 0,12 ml β-Propiolacton pro 100 ml Lösung bis zur pH-Konstanz behandelt. Nach einer Aktivkohlebehandlung wurde sterilfiltriert. Die erhaltene Lösung eignete sich für die intravenöse Applikation.

Beispiel 4

Präzipitat III der Rivanol-Ammonsulfat-Fraktionierung wurde in Wasser zu einer Lösung mit einer Proteinkonzentration von 3% gelöst. Durch Dialyse gegen 0,0005molaren Phosphatpuffer vom pH 6,2 wurde eine Euglobulinfällung durchgeführt. Nach Auflösung des Niederschlages in 0,9%iger Kochsalzlösung zu einer 5%igen Proteinlösung wurde mit 0,15 ml β-Propiolacton pro 100 ml Lösung bei pH 8,0 und 37 °C bis zur pH-Konstanz behandelt und sterilfiltriert. Die erhaltene Lösung eignete sich für die intravenöse Applikation.

**Patentansprüche**

1. Verfahren zur Herstellung einer für die intravenöse Applikation geeigneten Immunglobulinlösung durch Behandlung einer durch Fraktionierung aus Blutplasma oder Serum erhaltenen Proteinfraktion, die gegebenenfalls durch Behandlung mit kolloidaler Kieselsäure von Lipiden befreit wurde, mit β-Propiolacton bei Temperaturen von 20 bis 37 °C und pH-Werten von 7,0 bis 8,5 für die Dauer von 2 bis 10 Stunden bis zur pH-Konstanz, Aufarbeitung in bekannter Weise und Sterilfiltration, dadurch gekennzeichnet, dass man als Ausgangsmaterial eine Plasma- oder Serumproteinfraktion verwendet, die IgM in ankonzentrierter Form enthält, und das Verhältnis von β-Propiolacton zu einer 5%igen Lösung der IgM enthaltenden Proteine auf 0,05 bis 0,15 ml pro 100 ml einstellt.

2. Verfahren nach Anspruch 1, dadurch gekennzeichnet, dass man als Ausgangsmaterial eine Cohn-Fraktion III aus menschlichem Plasma verwendet, die in physiologischer Kochsalzlösung zu einer etwa 5%igen Proteinlösung gelöst wurde.

3. Verfahren nach Anspruch 2, dadurch gekennzeichnet, dass man die Proteinlösung vor der Behandlung mit β-Propiolacton mit Diethylaminoethylgruppen tragenden, quervernetzten Dextranen oder Cellulose, vorzugsweise unter der Handelsbezeichnung DEAE-Sephadex A-50 oder DEAE-Cellulose bekannten Anionenaustauschern, behandelt.

**Claims**

1. Process for the preparation of an immunoglobulin solution, suited for intravenous administration, by treating a protein fraction, obtained by fractionation from blood plasma or serum, optionally freed from lipids by treatment with colloidal silica gel, with β-propiolactone at temperatures of from 20 to 37 °C and pH-values of from 7.0 to 8.5 for about 2 to 10 hours until a constant pH-value is obtained, treating in a manner known per se and by sterile filtration, characterized in using as starting material a plasma or serum protein fraction containing IgM in concentrated form and adjusting the ratio of β-propiolactone to a 5% solution of the IgM containing proteins to 0.05 to 0.15 ml per 100 ml.

2. Process according to claim 1, characterized in using as starting material a Cohn fraction III of human plasma which has been dissolved in physiological saline solution to form an about 5% protein solution.

3. Process according to claim 2, characterized in treating the protein solution before the treatment with β-propiolactone by diethylaminoethyl groups-carrying crosslinked dextrans or cellulose, preferably by anion exchangers known by the trade names of DEAE-Sephadex A-50 or DEAE-Cellulose.

**Revendications**

1. Procédé de préparation d'une solution d'immunoglobulines convenant pour l'administration intraveineuse par traitement d'une fraction protéinique obtenue par fractionnement à partir de plasma sanguin ou de sérum, débarrassée le cas échéant des lipides par traitement avec de l'acide silicique colloïdal, par la β-propiolactone à une température de 20 à 37 °C et à un pH de 7,0 à 8,5 pendant 2 à 10 heures jusqu'à constance du pH, poursuite du traitement d'une manière connue et filtration stérile, caractérisé en ce qu'on utilise comme matière de départ une fraction protéinique du plasma ou du sérum qui contient de l'IgM sous forme concentrée et en ce que le rapport de la

β-propiolactone à une solution à 5% des protéines contenant de l'IgM est ajusté de 0,05 à 0,15 ml pour 100 ml.

2. Procédé selon la revendication 1, caractérisé en ce qu'on utilise comme matière de départ une fraction III de Cohn provenant de plasma humain qui a été dissoute dans une solution de chlorure de sodium physiologique jusqu'à l'obtention d'une solution à 5% environ de protéine.

3. Procédé selon la revendication 2, caractérisé en ce qu'on traite la solution protéinique avant le traitement par la β-propiolactone, par des dextranes réticulées portant des groupements diéthyl-aminoéthyles ou de la cellulose, de préférence des échangeurs d'anions connus sous le nom commercial de DEAE-Sephadex A-50 ou de DEAE-Cellulose.